# EUROPEAN PATENT APPLICATION

(11) **EP 4 656 226 A1**
(43) Date of publication of application: **03.12.2025**
(21) Application number: 24382588.2
(22) Date of filing: 31.05.2024
(51) Int. Cl.: A61M 21/00

(54) **A METHOD AND A SYSTEM FOR PERFORMING A SESSION OF STIMULATION OF FIVE SENSES**

(71) Applicant: El Punto Genuino, S.L., 28760 Tres Cantos, Madrid (ES); Multisensorial Training, S.L., Madrid (ES); Prisma Virtual Reality, SL, 28043 Madrid (ES)
(72) Inventor: ORTIZ ALONSO, Tomás, Madrid (ES); ZUASTI SUÁREZ, Iker, Madrid (ES)
(74) Representative: Balder IP Law, S.L.

(57) **Abstract**

The invention relates to a method for performing a session of stimulation of five senses A, B, C, D and E of a human user and to a system for performing the method. The session of stimulation comprises determined stimuli of the five senses and a determined stimulation time for each stimulus of the determined stimuli; the method comprises producing each stimulus of the determined stimuli at the respective stimulation time for the respective stimulus by actuating stimulators of the five senses; in first portions of a period of the session a simultaneous stimulation of A, B, C, D and E is produced; and in each portion of the period of the session between two consecutive first portions of the session: at least one of A, B, C and D is stimulated, and E is not simultaneously stimulated with the stimulated at least one of A, B, C and D.

## Description

### TECHNICAL FIELD

The present invention is encompassed within the field of systems and methods for stimulating senses of humans.

### BACKGROUND

Systems and methods for stimulating senses of a human with stimulators are known in the art. For example, equipment of virtual reality for stimulating several senses of a user (for example, sight, hearing and/or touch) is known in the art. In addition, several advantages of stimulation of senses are known in the art. However, said methods and systems can be further improved for enhancing brain development of users thereof.

### SUMMARY

A first aspect of the disclosure relates to a system for performing a session of stimulation of five senses A, B, C, D and E of a human user, the session of stimulation comprising determined stimuli of the five senses and a determined stimulation time for each stimulus of the determined stimuli;
the system comprising stimulators of A, B, C, D and E, the stimulators of A, B, C, D and E being: a head-attachable display for stimulating sight, a loudspeaker for stimulating hearing, a tactile stimulator, a scent emitter for stimulating smell and a taste stimulator;
the system comprising processing means configured to actuate each one of the stimulators at determined actuation times to produce each stimulus of the determined stimuli at the respective stimulation time;
wherein the processing means are configured to actuate the stimulators of A, B, C, D and E to produce a simultaneous stimulation of A, B, C, D and E in first portions of a period of the session; and
wherein the processing means are configured to actuate at least one of the stimulators of A, B, C and D to stimulate at least one of A, B, C and D in each portion of the period of the session between two consecutive first portions, and are not configured to actuate the stimulator of E to stimulate E simultaneously with the stimulated at least one of A, B,C and D in each portion of the period of the session between two consecutive first portions.

The five senses of a human user are sight, hearing, touch, smell and taste. Each one of the terms A, B, C, D and E refers to a different sense. For example, in some embodiments, A refers to sight, B to hearing, C to touch, D to smell and E to taste, whereas in other embodiments A refers touch, B refers to taste, C refers to hearing, D refers to sight and E refers to smell.

A session of stimulation refers to a period in which the five senses are stimulated in a determined manner (i.e., determined stimuli and determined stimulation times assigned to each stimulus of the determined stimuli), wherein the manner is preferably determined prior to starting the session of stimulation.

Each stimulus of the session is produced in the respective determined stimulation time. In other words, each stimulus of the determined stimuli is assigned a particular stimulation time within the period of the session, and the stimulus is to be produced at the assigned stimulation time. In this way the user is subjected to a sequence of stimuli along the session.

In some embodiments, the stimulation time of the determined stimuli is independent of the actions that a user of the system performs during the session. It has been determined that it is not required to adapt the stimuli of the session to how a user of the system has behaved previously in the same session to enhance brain development. In addition, this enables that the stimuli of the session are defined before the session starts, so that a less complicated preparation of the session is required (i.e., compared to a preparation that requires adapting the stimuli of the session to the user according to how the user has previously behaved in the same session).

A stimulus of an individual sense of a user stimulates a particular brain portion which responds to said stimulus. For example, a sight stimulus stimulates an occipital lobe, a hearing stimulus stimulates a temporal lobe, a touch stimulus stimulates a parietal lobe, a smell stimulus stimulates an entorhinal cortex, and a taste stimulus stimulates an insula.

In some embodiments, the determined stimuli comprise stimuli of different senses caused by a same real object. For example, the display shows a flower, the scent emitter emits a scent which smells like the flower. In another example, the display shows a sea, the loudspeaker emits sound of waves of a sea and the scent emitter emits scent which smells like a sea.

In some embodiments, the determined stimuli comprise showing a particular food on the display by actuating the head-attachable display and, at the same time, emitting a scent which smells like the shown food by actuating the scent emitter, and stimulating taste (e.g., providing a drop of water on the tongue) by actuating the taste stimulator.

The configuration of the processing means is for actuating the stimulators for producing the determined stimuli of the session at the determined stimulation time for each stimulus of the determined stimuli. The processing means may process instructions for actuating the stimulators.

The stimuli produced by the stimulators are the determined stimuli of the session.

The system may be configured to perform different sessions of stimulation (i.e., to perform sessions of stimulation comprising different stimuli and/or different stimulation times). Different sessions of stimulation allow stimulating the five senses in a different manner, thereby stimulating portions of the brain in a different manner. Performing different sessions is advantageous because a more complete stimulation of the brain is achieved compared to recurrently performing the same session.

In some embodiments, the system is configured to perform a subsequent session after waiting at least a time T from an end of a latest performance of a session. In this way, the system enables that the user rests before starting the subsequent session, so that stimulation of the brain changes compared to a stimulation of the brain that would happen if the subsequent session were performed immediately after the latest session. This rest time is advantageous for enhancing brain development because constant brain stimulation causes exhaustion of neurons and decreases brain attention to the stimuli. The rest time allows consolidating recently acquired knowledge and transforming a transitory memory into a lasting memory. Rest between sessions is a diffuse, non-conscious, relaxed way to maintain and enhance the learning performed in an active, attentive and event-focused way during the session. However, waiting too much time between consecutive sessions may decrease brain development due to the lack of the stimuli. For example, for sessions wherein each session has a period (i.e., duration) of at least one minute and at most sixty minutes, for example, at least ten minutes and at most forty-five minutes, it has been found advantageous for brain development to wait between three and thirty-six hours.

A user decreases attention to stimuli produced by the stimulators at an end of a session of more than sixty minutes, thus worsening brain development. It has been found that a session of less than one minute may cause worse brain development than a longer a session because one minute is little time to produce several consecutive simultaneous stimulations of five senses separated by appropriate stimulations of less than five senses.

The stimulator of sight is the head-attachable display, the stimulator of hearing is the loudspeaker, the stimulator of touch is the tactile stimulator, the stimulator of smell is the scent emitter, and the stimulator of taste is the taste stimulator.

Using a head-attachable display allows minimizing stimuli of sight caused by a real environment of the user.

In some embodiments, the loudspeaker is attachable to the ears of the user and is configured to minimize stimuli of hearing caused by a real environment of the user.

Minimizing stimuli caused by a real environment of a user is advantageous for enhancing control of the stimuli of the user during the session. In this sense, it is advantageous to use equipment for generating a virtual reality because this equipment allows maximizing isolation of the user from the real environment of the user.

The head-attachable display may be configured to, upon being actuated by the processing means, show images (e.g., a video) on the display. Upon wearing the head-attachable display, the images shown on the display can be viewed by the user, thereby stimulating sight of the user. The head-attachable display is, for example, virtual reality glasses (VR glasses).

The loudspeaker may be configured to, upon being actuated by the processing means, emit sounds (e.g., at least one of music and voices). To make the sound hearable by the user, the loudspeaker is close enough to the user (e.g., the loudspeaker may be part of earphones worn by the user) and emits sound frequencies audible by humans, for example, sound having a frequency of at least 500 Hz and at most 2000 Hz. A frequency of at least 500 Hz and at most 2000 Hz is a frequency of human voice used for communicating (i.e., for talking). Using a frequency of a human voice is advantageous because it allows reinforcing neural networks used in the processing human language. Aging may cause that sensory processing by the brain be worsened. Using a frequency of a human voice allows stimulating, and hence strengthening, neural networks used for processing the human voice. Thereby, allowing slowing the worsening of processing of sounds having a frequency within said range.

The tactile stimulator is configured to, upon being actuated by the processing means, stimulate touch in one or more body parts. For example, the tactile stimulator may be configured to stimulate touch in at least one of: hands, arms, neck, head and torso. Some examples of tactile stimulators are stimulators attached to means for mounting the stimulator to a respective part of the body. To stimulate the respective part of the body, the stimulator is moved (e.g., vibrates), while in contact with the respective body part. Said movement may be attained by consuming electrical energy.

The scent emitter is configured to, upon being actuated by the processing means, emit a scent. To make the scent smellable by the user, the scent emitter is close enough to the user.

Several stimulators may form part of a same device (e.g., the stimulator of sight and the stimulator of hearing may form part of a same device, and/or the tactile stimulator and the stimulator of sight may form part of a same device) or each stimulator may be in one or more separate devices.

The processing means comprise, for example, at least one of: one or more digital signal processors, one or more analogue/digital signal processing systems including field programmable gate arrays (FPGA), one or more standard processors (e.g., microprocessors) and one or more application specific signal processors (ASSP or ASIC). In some embodiments, the processing means may comprise several processing devices, and the stimulators are not actuated with the same processing device(s).

Each stimulus of the session is assigned an actuation time. To produce each stimulus S at the stimulation time assigned to the respective stimulus S, the stimulator for producing the stimulus S is actuated at the actuation time assigned to the stimulus S. The actuation time of the stimulus S is previous to the stimulation time of the stimulus S, and depends, at least in part, on the dynamics of the stimulator.

In addition to the dynamics of the stimulator, there are other parameters that may influence the exact time at which the stimulators are actuated, for example, processing speed and processing load of the processing means. Thereby, in some cases it may not be possible to establish an exact determined actuation time in advance to the session, and hence to establish an exact stimulation time. Thereby, each one of the actuation time and the stimulation time assigned to each stimulus may be a time range. Said time ranges may be implicitly defined in the sense that the configuration of the processing means does not need to explicitly define/state said time ranges. Instead, said time ranges may be implicitly defined, for example, by assuming a certain order of execution of instructions defined in the configuration, and a certain processing time for the execution of each instruction. Thereby, by placing the instructions in the correct order and including instructions requiring an appropriate execution time, the time ranges are implicitly defined.

Some stimulators (for example, a display, a loudspeaker and a tactile stimulator) normally have relatively low actuation times (i.e., delay of responses). However, others (for example, a taste stimulator) may have longer actuation times.

The session comprises first portions wherein A, B, C, D and E are stimulated at the same time. In the present disclosure, these portions are referred to as "first portions" for the mere sake of conciseness. Stimulating the five senses at the same time enhances neural development due to enhancing brain plasticity and strengthening neural connections. This stimulation enhances cognitive development, enhancing memory, concentration and sensory processing. Stimulating the five senses at the same time increases communication among frontal, parietal, occipital and temporal portions of the brain. Thereby enhancing efficiency of functional connectivity which allows enhancing and extending neural networks associated with the aforementioned brain portions to enhance the cognitive functions performed in said portions, and particularly enhancing neural networks for integrating the different brain portions. The simultaneous stimulation of the five senses allows enhancing a capability of decision making consistent with plasticity and stability of the neural networks. In addition, the simultaneous stimulation of the five senses allows enhancing synchronization and temporal stabilization of brain waves (e.g., theta waves, alpha waves and/or beta waves). The temporal stabilization of the brain waves allows enhancing temporal consistency of the sensory information.

The synchronization of the stimulation of the five senses allows receiving information of a particular event via different sensory routes of the brain, so that more details of the event are perceived compared to if less of the five senses are stimulated.

Between two consecutive first portions in which the five senses are simultaneously stimulated, the period of the session comprises a portion in which less than five senses are simultaneously stimulated (e.g., just one sense is stimulated). By not stimulating the five senses simultaneously but stimulating at least one sense, the system allows that a user does not lose continuity of perception with the reality generated by the system and allows preparing the brain for a subsequent simultaneous stimulation of the five senses (e.g. allows minimizing an overstimulation of the brain). For conciseness, in the present disclosure, each portion between two consecutive first portions is called a second portion.

With the stimulation according to the first aspect of the disclosure, the system allows slowing the aging process of a brain and even rejuvenating the brain. More specifically, it allows slowing: reduction of sensory sensitivity, worsening of reflex, worsening of movement problems, reduction of memory, reduction of neural connections, reduction of neurons and reduction of activity of neurons.

Since the system allows controlling which stimuli are included in the session and the timing of said stimuli, each simultaneous stimulation of the five senses can be configured according to a particular aim. The particular aim may be that the user performs certain physical actions. As information reaches a portion of the brain, known as motor region, this region causes that the user moves in a particular manner (i.e., performs certain actions).

In some embodiments, the determined stimuli of the session comprises instructions for the user, the instructions being for using a sense in a determined manner in relation to an stimulus of the determined stimuli, the stimulus being different than instructions for the user; the system being configured to actuate an stimulator to produce, when the instructions for the user have ended, the stimulus different than instructions for the user.

Thereby, the system allows that a user interacts with a stimulus of the stimuli in a desired manner. The fact that the stimulus is produced when the instructions have ended does not mean that the stimulus does not start earlier. For example, the system can be configured to actuate the stimulator to start to produce the stimulus before the instructions. In this way, the stimulus is not produced too late after the instructions even if a required processing time and/or the actuation time of the stimulator is longer than initially expected.

In some embodiments, the instructions are at least one of: instructions related to the sense of sight, instructions related to the sense of hearing, instructions related to the sense of touch, instructions related to the sense of smell and instructions related to the sense of taste.

In some embodiments, the instructions related to the sense of sight are instructions for focusing visual attention on a particular visual feature (e.g., a sea or a flower) shown on the display.

In some embodiments, the instructions related to the sense of hearing are instructions for focusing on a sound emitted by the loudspeaker (e.g., sound of sea waves, sound of birds chirping and/or music).

In some embodiments, the instructions related to the sense of touch are instructions for focusing on a particular touch stimulus (e.g., a touch on an arm).

In some embodiments, the instructions related to the sense of smell are at least one of: focus on a smell of a scent emitted by the scent emitter, inhaling, holding breath, exhaling, inhaling during a particular time, holding breath during a particular time, exhaling during a particular time, slowly inhaling, and taking a deep breath.

In some embodiments, the instructions related to the sense of taste are focusing on a flavor of a particular food.

In some embodiments, the stimulus different than the instructions for the user is comprised within the stimuli of different senses caused by a same real object. Thereby the same real object is perceived by several senses, as a user would do in the real world.

In some embodiments, the tactile stimulator comprises vibrating devices for being attached to a human body, and the system is configured to cause that the vibrating devices vibrate at a frequency of at least 3.5 Hz and at most 7.5 Hz.

A frequency of at least 3.5 Hz and at most 7.5 Hz is a frequency of a theta wave. Theta waves stimulate certain portions of the brain to promote learning. It has been observed that vibration of the vibrating devices within the frequency range of at least 3.5 Hz and at most 7.5 Hz stimulate said portions of the brain with theta waves to promote learning.

In some embodiments, the vibrating devices are for being attached to the pinna. Attaching the vibrating devices to the pinna is advantageous for enhancing transmission of the vibration frequency to relevant portions of the brain for the following reasons: the shape of the ear allows amplifying the vibrations of the vibrating devices, and the pinna is an anatomic structure related to portions of the brain related to language (e.g., portions of the limbic system, such as, hippocampus and amygdala) where theta waves are also present (e.g., hippocampal theta).

In some embodiments, the vibrating devices are for being attached to at least one of a tragus, a cavum conchae, a cymba conchae and an antihelix of an ear. These locations enable further improvement of the effect of the vibration frequency on relevant portions of the brain compared to other locations of the ear, thus enhancing stimulation of said brain portions with theta waves. This effect is further enhanced if at least one vibrating device is attached in each one of the tragus, the cavum conchae, the cymba conchae and the antihelix. For example, the tactile stimulator may comprise a first vibrating device for being attached to the tragus, a second vibrating device for being attached to the cavum conchae, a third vibrating device for being attached to the cymba conchae and a fourth vibrating device for being attached to the antihelix of the ear.

In some embodiments, the system is configured to cause that the vibrating devices vibrate in phase and at a frequency of at least 3.5 Hz and at most 7.5 Hz. Vibration in phase of the vibrating devices means that all the vibrating devices have the same phase of vibration wave at every time. Vibrating in phase allows enhancing stimulation of the brain with theta waves because of constructive interference of the vibration.

In some embodiments, the vibrating devices are attached to an ear-attachable support body. An ear-attachable support body facilitates attachment of the vibrating devices to suitable locations of the ear, since all the vibrating devices may be attached to the ear by attaching the support body to the ear (i.e., it is not required to separately attach each vibrating device to the ear). A person skilled in the art is aware of several ways of implementing bodies being ear-attachable, e.g., a body having a suitable shape complementary to a shape of a portion of the ear or a body having a band worn on the head (e.g., resembling a headphone).

In some embodiments, the ear-attachable support body comprises a portion for at least partially covering the tragus, a portion for at least partially covering the cavum conchae, a portion for at least partially covering the cymba conchae and a portion for at least partially covering the antihelix; and there is at least one vibrating device in each one of said portions of the ear-attachable support body. In this way, when the ear-attachable support body is attached to the ear, there is at least one vibrating device in contact with each one of the tragus, the cavum conchae, the cymba conchae and the antihelix for transmitting vibration to the respective portion of the ear.

In some embodiments, each vibrating device has a size that allows the vibrating device to be housed in a parallelepiped having a first side of less than 20 mm, a second side of less than 10 mm, and a third side of less than 5 mm, such as a first side of less than 15 mm, a second side of less than 5 mm and a third side of less than 2 mm.

In some embodiments, each vibrating device is a piezoelectric device. A piezoelectric device can be suitable for performing vibrations at a frequency of at least 3.5 Hz and at most 7.5 Hz and can be made small enough to be attached to particular portions of the pinna of the ear (e.g., the tragus, the cavum conchae, the cymba conchae and/or the antihelix).

In some embodiments, the processing means are configured to control vibration frequency of the vibrating devices. For example, the processing means may be configured to control vibration frequency and intensity of the vibrating devices (e.g., piezoelectric devices) by using suitable audio signals. Thereby, the vibration frequency and intensity are dependent on the audio signals. In particular, an electrical energy dependent on the audio signal may be applied to the piezoelectric device, thus causing a deformation of the piezoelectric device for vibrating with an intensity and frequency based on the audio signal.

In some embodiments, the processing means are configured to:
- actuate the vibrating devices for vibration at a frequency of at least 3.5 Hz and at most 7.5 Hz, and
- separately actuate other devices of the tactile stimulator (e.g., a vest for stimulating a torso at a frequency outside of the range of at least 3.5 Hz and at most 7.5 Hz).

In this way, the processing means do not necessarily actuate the vibration having a frequency of at least 3.5 Hz and at most 7.5 Hz whenever the vibration of the other devices is actuated and vice versa. Similarly, the processing means may be configured to separately stop the actuation.

Although it has been previously described that the tactile stimulator may comprise vibrating devices for just one ear, the tactile stimulator may comprise vibrating devices for the other ear. Providing vibrating devices to both ears allows enhancing stimulation of the brain portions with theta waves. The features mentioned in relation to the vibrating devices of one ear are also applicable to the vibrating devices of the other ear.

In some embodiments, the processing means comprise a control circuit for actuating vibration of the vibrating devices and for actuating the taste stimulator. This allows facilitating a coordinated control (e.g., synchronization) of the vibrating devices and the taste stimulator because both are actuated by the same control circuit. In addition, this allows simplifying cables and space required for the system since a same control circuit is shared by both actuators.

In some embodiments, the system is configured to cause that the head-attachable display shows light pulses on the display, the light pulses having a frequency of at least 3.5 Hz and at most 7.5 Hz (i.e., at least 3.5 pulses per second and at most 7.5 pulses per second).

Pulsed light having a frequency of at least 3.5 Hz and at most 7.5 Hz allows stimulating the brain with theta waves, which promote learning.

Showing the light pulses in a periphery of the display is advantageous for minimizing disruption of an intended sight stimulus (i.e., disruption of the image(s) that the user should view) compared to pulses of light shown, e.g., in a center of the display.

In some embodiments, the light pulses have a same color. Light pulses of a same color allow further enhancing stimulation of the brain.

In some embodiments, each light pulse of the light pulses has a same duration. Having a same duration increases a degree of similarity among the light pulses, thus increasing a recurrence degree of the light pulses, thereby enhancing the stimulation with theta waves.

In some embodiments, the color of the light pulses is the same for all light pulses. Having a same color increases a degree of similarity among the light pulses, thus increasing a recurrence degree of the light pulses, thereby enhancing the stimulation with theta waves.

In some embodiments, the color of the light pulses is a color which is not included in a periphery of images shown in the session. The periphery of the images shown in the session may have a color (i.e., when disregarding the light pulse) which is similar to a color of the light pulse. If this is the case, a frequency of the light pulse perceived by a user of the system may be different that the frequency of the light pulses. By using a color different than every color of periphery of the images, said undesirable change of perceived frequency is prevented.

In some embodiments, the color of the light pulses is different than a color that appears the most in the periphery of the images of the session, thus the undesirable change of perceived frequency may be minimized because at least the color that appears the most in the periphery of the images of the session will show a contrast with the color of the light pulses. The color that appears the most may be the color that has a greater value calculated as the sum of the areas having said color and located in a periphery of the images of the session.

In some embodiments, a color of the light pulses is white. It has been determined that in most images that depict reality (e.g., a forest or a sea) a content of white in the periphery of the images is normally low, so that, when using images that depict a reality, white has high chances that a user perceives the intended frequency of the light pulses of at least 3.5 Hz and at most 7.5 Hz without requiring to analyze the color of the periphery of all images.

In some embodiments, the light pulses are superposed to images of a video shown on the display. The light pulses look like, for example, part of a head-up display between the head-attachable display and an eye of the user.

It is not required that the periphery of the display be black between consecutive light pulses to be able to show the light pulses. The display may show more than one image between consecutive pulses, so that color of the periphery may change between two consecutive pulses.

In some embodiments, the light pulses are shown in a lateral periphery or at opposite lateral peripheries of the display, and the light pulses are not shown in a top periphery and in a bottom periphery of the display. The terms "lateral", "top" and "bottom" refer to a position of the head-attachable display when attached to the head of an upright user. Opposite lateral peripheries of the display refer to a right periphery of the display and to a left periphery of the display.

Not showing the light pulses in the top periphery and/or in the bottom periphery is advantageous because it allows minimizing a display space required to show the light pulses while allowing stimulating the brain with theta waves.

In some embodiments, stimulation in the first portions of the period of the session comprises simultaneous stimulation:
- with the vibrating devices,
- with other components of the tactile stimulator (e.g., vibration of a vest and/or of other components attachable to arms and/or attachable to legs),
- with the pulses of light, and
- with images shown on the display.

In this way the five senses are simultaneously stimulated and, in addition, a simultaneous stimulation with the pulses of light and the vibrating devices at one or more frequencies of at least 3.5 Hz and at most 7.5 Hz is produced. Performing said simultaneous stimulation enhances learning during stimulation of the five senses because the brain is simultaneously stimulated with the theta waves due the frequencies of at least 3.5 Hz and at most 7.5 Hz of the pulses of light and of the vibration of the vibrating devices. Thereby, allowing further enhancing brain development.

In some embodiments, the taste stimulator comprises a conduit with an open end, the conduit being for conveying an edible substance; the open end being for exit of an edible substance and being introduceable in a mouth of a human user; the taste stimulator comprises a container for an edible substance, the conduit being connected to an interior of the container; the taste stimulator comprises means for moving an edible substance from the interior of the container to the open end of the conduit via the conduit.

The open end is introduceable (i.e., is suitable for being introduced) in a mouth of a human user.

The taste stimulator is configured to, upon being actuated by the processing means, introduce an edible substance into the mouth of the user by using the means for moving an edible substance.

The means for moving an edible substance comprise, for example, a pump. In some embodiments, the edible substance is a semisolid or liquid (e.g., liquid water).

In some embodiments, the means for moving an edible substance is a micropump. A micropump is suitable for moving relatively small amounts of edible substance (e.g., just some drops of a liquid) which is enough for stimulating taste.

In some embodiment, the conduit has a shape of a straw.

Providing a conduit enhances comfort of the user of the taste stimulator because the conduit minimizes a need for manipulating the container for ensuring that edible substance enters the mouth after exiting the taste stimulator. In some embodiments, it may be sufficient that the user manipulates the conduit, not requiring manipulation of the container. In addition, the container may be kept at a distance from the user while using the taste stimulator.

In some embodiments, the system is configured to have the container above the open end of the conduit when the taste stimulator is in a use position. A container above the open end is advantageous for assisting in the movement of the edible substance from the container to the open end of the conduit. In this way, a delay of response of the taste stimulator can be minimized.

In some embodiments, the taste stimulator is attached to the head-attachable display (e.g., to the VR glasses), and the open end of the conduit of the taste stimulator is below the container of the taste stimulator. In this way, the taste stimulator is attached to the head-attachable display to place the container of the taste stimulator above the open end of the conduit.

In some embodiments, the system is configured to include, by using the means for moving an edible substance, an edible substance within the conduit when the processing means actuate the taste stimulator.

A delay of response of the taste stimulator (i.e., a delay between processing actuation instructions to move edible substance through the open end of the conduit and the performance of said movement) may be longer than the delay of response of at least one of the other stimulators. In addition, the delay of response of the taste stimulator may vary during use, which could cause errors of synchronization of taste stimuli with stimuli of other senses of a session.

Keeping edible substance within the conduit allows decreasing the delay of response compared to having to provide the edible substance directly from the container instead of from the conduit. In addition, keeping edible substance within the conduit allows decreasing the variability of the delay of response. A variability of the response is due to the need for filling a volume of the container prior to conveying the edible substance through the conduit. Said filling cause variability which depends, among other factors, on an amount of edible substance within the container, a location of the edible substance within the container and shape of the container. By keeping edible substance within the conduit it is made sure that, when the processing means actuate the taste stimulator, the edible substance has already entered the conduit, so that it is not required that the edible substance is moved to fill the volume of the container prior to entering the conduit.

In some embodiments, the system is configured to completely fill the conduit, by using the means for moving an edible substance, with an edible substance when the processing means actuate the taste stimulator. Completely filling the conduit allows minimizing a delay of a response of the taste stimulator because the edible substance is already as close as possible to the open end of the conduit when the taste stimulator is actuated.

Different configurations of the system enable that the conduit contains an edible substance when the processing means actuate the taste stimulator.

In some embodiments, the system is configured to move edible substance from the container into the conduit before starting the session or at a portion of the session prior to the first actuation of the taste stimulator. Thereby, the edible substance is provided to the conduit prior to the first actuation of the taste stimulator.

In some embodiments, the system is configured to move edible substance from the container into the conduit while instructions for placing the taste stimulator are being provided. In this way, it is allowed minimizing a time required for preparing the system for use because the container is filled at the same time as the user is provided with the instructions for placing the taste stimulator.

In some embodiments, the system may be configured to keep edible substance within the conduit after actuating the taste stimulator, for example, by providing means that block a return of the edible substance from the conduit into the container. For example, for providing an edible substance out of the open end of the conduit, the means for moving an edible substance may introduce more edible substance into the conduit than a maximum amount which can be contained in the conduit, so that some of the edible substance must leave the conduit through the open end and some of the edible substance does not leave the conduit. Thereby, keeping some of the edible substance within the conduit after edible substance exits the conduit allows enabling that, in the next actuation of the taste stimulator, the conduit will already contain edible substance.

In some embodiments, a dispenser is within the conduit, for example, a one-way dispenser is within the conduit. Providing a one-way dispenser enables that an edible substance goes through the dispenser in one direction and minimizes edible substance which goes through the one-way dispenser in the opposite direction. In this way, because edible substance that goes in the opposite direction is minimized, it is enabled that the conduit keeps filled.

A user may be tempted to suck edible substance from the conduit, which may be undesirable because the stimuli produced could undesirably change and also because the conduit may be undesirably emptied. Providing a dispenser allows minimizing an amount of edible substance sucked from the conduit.

The force applied to make the edible substance leave the conduit may be at least one of a gravity force of the weight of the edible substance, lack of vacuum within the conduit and a force from a pump that pushes the edible substance out of the conduit.

In some embodiments, the system is configured to detect that the conduit does not contain edible substance and:
- upon detecting that the conduit does not contain edible substance, the means for moving an edible substance move edible substance from the container into the conduit; and
- upon detecting that the conduit contains edible substance, the means for moving an edible substance do not move edible substance from the container into the conduit.

In some embodiments, the system is configured to detect that the conduit does not contain edible substance and:
- upon detecting that the conduit does not contain edible substance and that the session comprises a taste stimulus at a later stage of the session, the means for moving an edible substance move edible substance from the container into the conduit;
- upon detecting that the conduit does not contain edible substance and that the session does not comprise any taste stimulus at a later stage of the session, the means for moving an edible substance do not move edible substance from the container into the conduit; and
- upon detecting that the conduit contains edible substance, the means for moving an edible substance do not move edible substance from the container into the conduit.

Thereby, the conduit is provided with edible substance when the conduit does not contain edible substance and there is a taste stimulus which will be produced at a later stage of the session. It is not required to provide an edible substance to the conduit if the session does not comprise any taste stimulus at a later stage of the session because taste will not be further stimulated in the session.

In some embodiments, the system is configured to provide, prior to starting a session, instructions to a user of the system, the instructions being for placing the vibrating devices in one or two ears of the user; the system being configured to provide the instructions by at least one of:
- showing, on the head-attachable display, the instructions for placing the vibrating devices, and
- emitting, by the loudspeaker, sound comprising instructions for placing the vibrating devices.

In some embodiments, the system is configured to provide, prior to starting a session, instructions to a user of the system, the instructions being for placing inside a mouth an open end of the taste stimulator, the open end being for exit of an edible substance; the system being configured to provide the instructions by at least one of:
- showing, on the head-attachable display, the instructions for placing the taste stimulator, and
- emitting, by the loudspeaker, sound comprising instructions for placing the taste stimulator.

By providing the instructions using the display and/or the loudspeaker of the system prior to starting the session, the user is made aware that the taste stimulator must be correctly placed prior to providing an edible substance out of the open end of the conduit.

In some embodiments, the system comprises one or more proximity sensors for detecting proximity between the taste stimulator and at least one of the head-attachable display, the loudspeaker and the tactile stimulator (e.g., an upper portion of a vest of the tactile stimulator). For example, the taste stimulator may be attached to a radiofrequency emitter, and at least one of the head-attachable display, the tactile stimulator and the loudspeaker may be attached to a radiofrequency receiver for receiving a radiofrequency emitted by the radiofrequency emitter. A distance between the receiver and the emitter may be estimated according to the power of the radiofrequency received, by the receiver, from the emitter (i.e., more power means closer, and less power means further). If the power received is greater than a threshold, it is estimated (for example, by the processing means of the system) that the taste stimulator is close enough to the head-attachable display, the tactile stimulator and/or the loudspeaker (and hence to a user wearing the respective stimulator) for the user to introduce the open end of the conduit in the mouth. In some embodiments, the receiver is attached to the taste stimulator instead of to the head-attachable display, the tactile stimulator and/or the loudspeaker; and the emitter is attached to the head-attachable display, the tactile stimulator and/or the loudspeaker instead of to the taste stimulator.

In some embodiments, the provision of instructions for placing inside a mouth an open end of the taste stimulator comprises:
- upon detecting that the power received is lower than the threshold, requesting the user to move the taste stimulator closer to the head of the user; and
- upon detecting that the power received is greater than the threshold, stopping requesting the user to move the taste stimulator closer to the head of the user.

In some embodiments, a delay of a response of the taste stimulator is of at least 0.2 seconds and at most 4 seconds, for example, of at least 0.5 seconds and at most 2 seconds. It has been found that some actuators of taste stimulators (e.g., a taste stimulator in which the means for moving an edible substance is a micropump for moving liquid) have said delay of response.

In some embodiments, the processing means are configured to actuate the taste stimulator prior to actuating the other stimulators so that a simultaneous stimulation of the five senses is produced. Since the taste stimulator has delay of response greater than a delay of response of the other actuators, the instructions for producing the taste stimulus are processed prior to the processing of the respective instructions of the rest of the stimulators.

In some embodiments, stimulation of taste is produced by moving, out of the taste stimulator, at least 0.01 ml and at most 2 ml of edible substance, for example, of at least 0.02 ml and at most 1 ml, for example, of at least 0.03 ml and at most 0.5 ml. Providing a small amount of edible substance (e.g., water) into the mouth has been found enough to stimulate taste to provide suitable stimulation of the brain and to avoid overstimulating the brain.

In some embodiments, the processing means are configured to leave a time of at least thirty seconds between two consecutive actuations of the taste stimulator. Thereby, a session leaves a time of at least thirty seconds between two consecutive events wherein taste is stimulated. It has been found that thirty seconds is a minimum time required to minimize overstimulation of the sense of taste. If two stimulations are provided in less than thirty seconds a risk that a user does not detect the latest stimulation increases compared to leaving a greater time between consecutive stimulations because it is possible that the user does not detect the latest output of edible substance. For example, if the taste stimulator provides some drops of water just after having provided some drops of water, it is possible that the drops of water provided the latest are not detected by the sense of taste because they fall on the previously deposited drops of water.

In some embodiments, the system is configured to provide, prior to starting a session, instructions to a user of the system, the instructions being for placing the scent emitter near the user; the system being configured to provide the instructions by at least one of:
- showing, on the head-attachable display, the instructions for placing the scent emitter near the user, and/or
- emitting, by the loudspeaker, sound comprising the instructions for placing the scent emitter near the user.

By providing the instructions using the display and/or the loudspeaker of the system prior to starting the session, the user is made aware that the scent emitter must be correctly placed prior to emitting a scent.

In some embodiments, the system comprises one or more proximity sensors for detecting a proximity between the scent emitter and at least one of the head-attachable display, the loudspeaker and the tactile stimulator (e.g., an upper portion of a vest of the tactile stimulator). For example, the scent emitter may be attached to a radiofrequency emitter, and at least one of the head-attachable display, the tactile stimulator and the loudspeaker may be attached to a radiofrequency receiver for receiving a radiofrequency emitted by the radiofrequency emitter. A distance between the receiver and the emitter may be estimated according to the power of the radiofrequency received, by the receiver, from the emitter (i.e., more power means closer, and less power means further). If the power received is greater than a threshold, it is estimated (for example, by the processing means of the system) that the scent emitter is close enough to the head-attachable display, the tactile stimulator and/or the loudspeaker (and hence to a user wearing the respective stimulator) for the user to introduce the open end of the conduit in the mouth. In some embodiments, the receiver is attached to the scent emitter instead of to the head-attachable display, the tactile stimulator and/or the loudspeaker; and the emitter is attached to the head-attachable display, the tactile stimulator and/or the loudspeaker instead of to the scent emitter.

In some embodiments, the provision of instructions for placing inside a mouth an open end of the taste stimulator comprises:
- upon detecting that the power received is lower than the threshold, requesting the user to move the scent emitter closer to the user; and
- upon detecting that the power received is greater than the threshold, stopping requesting the user to move the scent emitter closer to the user.

In some embodiments, the processing means are configured to leave a time of at least thirty seconds between two consecutive actuations of the scent emitter.

Thereby, a session leaves a time of at least thirty seconds between two consecutive events wherein smell is stimulated. It has been found that thirty seconds is a minimum time required to minimize overstimulation of the sense of smell. If two stimulations are provided in less than five seconds a risk that a user does not properly detect the latest stimulation increases compared to leaving a greater time between consecutive stimulations because it is possible that the prior scent has not dissipated sufficiently.

In some embodiments, the processing means are configured to leave a time of at least thirty seconds between two consecutive actuations of the scent emitter; the processing means being configured to actuate the taste stimulator to stimulate taste simultaneously with stimulation of smell produced by the latest actuation of the two consecutive actuations of the scent emitter.

By waiting a time of at least thirty seconds between two consecutive actuations of the scent emitter, it is left time to dissipate the smell produced in the earlier actuation of the two consecutive actuations, so that smell produced in the later actuation of the two consecutive actuations is less affected by the smell produced in the earlier actuation of the two consecutive actuations. Since smell has an impact on taste, and taste is stimulated simultaneously with the stimulation of smell in the later actuation of the two consecutive actuations, it is decreased an effect on taste caused by the smell produced in the earlier actuation.

In some embodiments, the determined stimuli comprise instructions for the user to perform a movement of a part of the body of the user.

In some embodiments, the session comprises showing several images on the display, wherein some images of the several images have been shown on the display during the session and other images of the several images have not been shown on the display during the session; and some images of the images shown on the display during the session have been shown while simultaneously stimulating five senses of the user; wherein the processing means are configured to provide instructions for the user to determine, for each image of the several images, if the respective image has been previously shown on the display during the session.

By instructing the user to determine whether an image has been shown during the session, a function of a brain of the user (e.g., memory and/or attention) is evaluated by determining how many images are correctly determined by the user as images that have been previously shown in the session.

To evaluate evolution of biomedical parameters of a user, the following parameters of the user may be measured before the session and after the session: mobility, disturbances of speech, gait, hydration, skin color, body temperature, pulse, respiratory rate, blood pressure, height, weight, index of body mass, fat percentage, fat reserve, bone mass, residual mass, weight index, waist/hip ratio, biochemical parameters (e.g., glucose, cholesterol), endocrinological variables (ghrelin, leptin, insulin, sex hormones, cortisol, catecholamines, prolactin, growth hormone (GH), thyroxine), balance, muscle strength, muscle tension and grip strength.

To evaluate evolution of biomedical parameters of a user, the following parameters of the user may be measured before the session, during the session and after the session: electrodermal activity, degree of skin conductance, number of daily steps, respiratory rate, heart rate, variability of heart rate, sleep detection, daily temperature and oxygen level in blood.

A second aspect of the disclosure relates to a method for performing a session of stimulation of five senses A, B, C, D and E of a human user, the session of stimulation comprising determined stimuli of the five senses and a determined stimulation time for each stimulus of the determined stimuli;
the method comprising producing each stimulus of the determined stimuli at the respective stimulation time for the respective stimulus by actuating stimulators of the five senses, wherein in first portions of a period of the session a simultaneous stimulation of A, B, C, D and E is produced, and
wherein in each portion of the period of the session between two consecutive first portions of the session: at least one of A, B, C and D is stimulated, and E is not simultaneously stimulated with the stimulated at least one of A, B, C and D.

Similar advantages as those described with respect to the first aspect of the disclosure are also applicable to the second aspect of the disclosure.

### BRIEF DESCRIPTION OF THE DRAWINGS

To complete the description and to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:
Figure 1 shows a system in accordance with embodiments.
Figure 2 shows a diagram of a method in accordance with embodiments.
Figure 3 shows an ear of a human.
Figure 4 schematically shows a taste stimulator in accordance with embodiments.
Figure 5 shows a time evolution of a session in accordance with embodiments.

### DETAILED DESCRIPTION

The following description is not to be taken in a limiting sense but is given solely for the purpose of describing the broad principles of the invention. Embodiments of the invention will be described by way of example, with reference to the above-mentioned drawings.

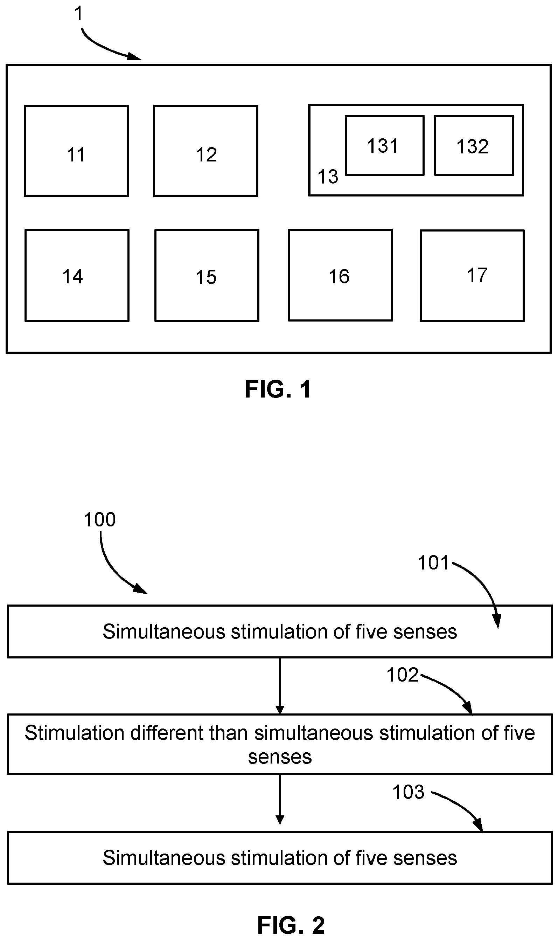

Figure 1 shows a system 1 for performing a session of stimulation of five senses A, B, C, D and E of a human user.

Figure 2 shows a method 100 for performing a session of stimulation of five senses A, B, C, D and E of a human user. The method 100 can be performed with the system 1.

The system 1 comprises stimulators of five senses of a human user. The stimulators are: a head-attachable display 11, a loudspeaker 12, a tactile stimulator 13, a scent emitter 14 and a taste stimulator 15. The system further comprises processing means 16 and a data storage 17.

The stimulators can be part of a set for generating a virtual reality for the user of the system 1.

The head-attachable display 11 is, for example, VR glasses. The loudspeaker 12 is, for example, a headphone.

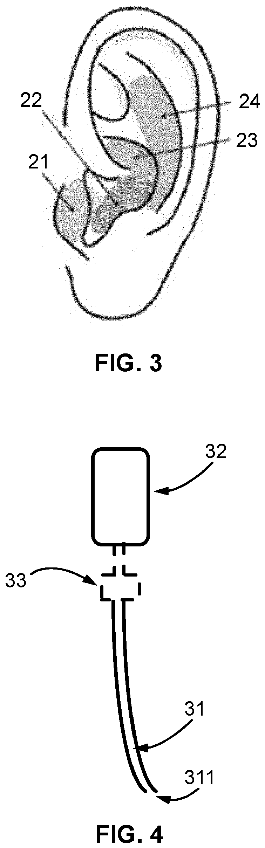

The tactile stimulator 13 comprises, for example, a haptic suit 131. The haptic suit 131 may comprise components, e.g., a vest and/or components attachable to arms and/or components attachable to legs. The components of the haptic suit 131 may be configured to vibrate. In addition to the haptic suit 131, the tactile stimulator 13 may comprise vibrating devices 132. The vibrating devices 132 of the tactile stimulator 13 may be for being placed on the following portions of an ear shown in Figure 3: a tragus 21, a cavum conchae 22, a cymba conchae 23 and an antihelix 24. For example, the system may comprise one vibrating device for each one of the portions 21, 22, 23 and 24 of the ear. The system may be configured to cause that the vibrating devices 132 vibrate at a frequency of at least 3.5 Hz and at most 7.5 Hz.

The scent emitter 14 is, for example, a device that can emit a scent from a variety of scents having different smells.

As shown in Figure 4, the taste stimulator 15 may comprise a conduit 31 with an open end 311, the conduit 31 being for conveying an edible substance; the open end 311 being for exit of an edible substance and being introduceable in a mouth of a human user; wherein the taste stimulator 15 comprises a container 32 (e.g., a bag or a box) for containing an edible substance, the conduit 31 being connected to an interior of the container 32; the taste stimulator 15 comprising means 33 for moving an edible substance from the interior of the container 31 to the open end 311 of the conduit via the conduit. The means 33 for moving an edible substance may comprise a micropump. The conduit 31 may be connected to the interior of the container 32 via the means 33 for moving an edible substance.

The processing means 16 are communicatively connected to the stimulators 11, 12, 13, 14, 15 and to the data storage 17. The processing means 16 may receive instructions (e.g., from the data storage 17) to be executed for performing a session of stimulation. The instructions may comprise instructions for actuating each stimulator of the stimulators at certain times.

Prior to starting a session of stimulation, the system 1 may provide instructions for correctly placing at least some of the stimulators 11, 12, 13, 14,15. For example, the display and/or the loudspeaker may provide instructions for the user to place the tactile stimulator 13, the scent emitter 14 and/or the taste stimulator 15 at an appropriate position.

The method 100 shown in Figure 2 is a method for performing a session of stimulation of five senses A, B, C, D and E of a human user, the session of stimulation comprising determined stimuli of the five senses and a determined stimulation time for each stimulus of the determined stimuli; the method comprises producing each stimulus of the determined stimuli at the respective stimulation time for the respective stimulus by actuating stimulators of the five senses, wherein in first portions of a period of the session a simultaneous stimulation of A, B, C, D and E is produced, and wherein in each portion of the period of the session between two consecutive first portions of the session: at least one of A, B, C and D is stimulated, and E is not simultaneously stimulated with the stimulated at least one of A, B, C and D.

Since the system 1 is configured to perform the method 100, the processing means 16 are configured to actuate each one of the stimulators 11, 12, 13, 14, 15 at determined actuation times to produce each stimulus of the determined stimuli at the stimulation time of the respective stimulus; the processing means are configured to actuate the stimulators of A, B, C, D and E to produce a simultaneous stimulation of A, B, C, D and E in first portions of a period of the session; and the processing means are configured to actuate at least one of the stimulators of A, B, C and D to stimulate at least one of A, B, C and D in each portion of the period of the session between two consecutive first portions, and are not configured to actuate the stimulator of E to stimulate E simultaneously with the stimulated at least one of A, B,C and D in each portion of the period of the session between two consecutive first portions. For conciseness, in the present disclosure, each portion of the period of the session between two consecutive first portions is referred to as a second portion.

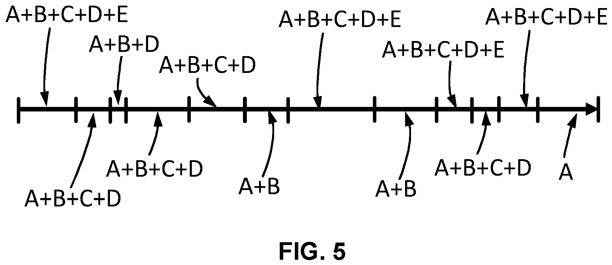

Figure 5 shows a horizontal timeline of events generated in a session of stimulation. In Figure 5, the timeline has been divided into portions, and each portion of the timeline has been linked to senses that are simultaneously stimulated in the respective portion. In particular, the following sequence of stimulations is generated according to the timeline from beginning to end: event 1: A+B+C+D+E, event 2: A+B+C+D, event 3: A+B, event 4: A+B+C+D, event 5: A+B+C+D, event 6: A+B, event 7: A+B+C+D+E, event 8: A+B, event 9: A+B+C+D+E, event 10: A+B+C+D, event 11: A+B+C+D+E and event 12: A.

Therefore, the five senses are simultaneously stimulated in events 1, 7, 9 and 11, and hence the portions of the sessions in which these events are generated are referred to as the first portions of the session. In the second portions of the session, the following events are generated: event 2, event 3, event 4, event 5, event 6, event 8 and event 10.

Since the session defines events in which the five senses are simultaneously stimulated 101, 103 and these events are separated by events in which stimulation different than simultaneous stimulation of five senses 102 (i.e., stimulation of at least one sense and of at most four senses simultaneously) is generated, the session is performed according to the method 100.

In at least some portions of the session, the display 11 may show light pulses in a periphery of the display, the light pulses having a frequency of at least 3.5 Hz and at most 7.5 Hz.

It is explained below an example of a session to which the timeline of Figure 5 could refer.

In the event 1 the following stimuli are simultaneously produced: the display shows images of a forest, the headphones emit sounds of a forest (e.g., bird sounds, wind sounds and/or sounds of movement of leaves of trees) , the vibrating devices vibrate at a frequency of 4 Hz, light pulses are shown in the periphery of the device at a frequency of 4 Hz, all the components of the haptic suit are actuated (e.g., vibrate), the scent emitter emits a smell of a forest, and between one and five drops of water go out of the open end 311 of the conduit of the taste stimulator. Event 1 also includes provision of instructions for the user during the event 1 (the instructions are completely provided prior to ending the event 1, and the duration of event 1 is sufficient to perform said instructions during event 1). The instructions are provided via sound emitted by the loudspeakers and comprise instructions to focus on the stimulation of the senses, to inhale during five seconds, then to hold breath during one second, and then to exhale during five seconds.

In the event 2, the following stimuli are simultaneously produced: the display shows a rose, the vibrating devices vibrate at a frequency of 4 Hz, light pulses are shown in the periphery of the device at a frequency of 4 Hz, all the components of the haptic suit are actuated (e.g., vibrate), and the scent emitter emits a smell of a rose. The event 2 also includes provision of instructions for the user. The instructions are for smelling the rose and are provided via sound emitted by the loudspeaker.

In the event 3, the following stimuli are simultaneously produced: the display shows a geranium, and the scent emitter emits a smell of a geranium. The event 3 also includes provision of instructions for the user. The instructions are for smelling the geranium and are provided via sound emitted by the loudspeaker.

In the event 4, the following stimuli are simultaneously produced: an image of the younger user is shown at least subliminally, the vibrating devices vibrate at a frequency of 4 Hz, light pulses are shown in the periphery of the device at a frequency of 4 Hz, all the components of the haptic suit are actuated (e.g., vibrate), and the scent emitter emits a smell. Event 4 also includes provision of instructions for the user. The instructions are for breathing deeply and slowly, and for standing up on one leg and subsequently standing up on the other leg; the instructions are provided via sound emitted by the loudspeaker.

In the event 5, the following stimuli are simultaneously produced: an image of the user at an age younger than his/her current age is shown at least subliminally, the vibrating devices vibrate at a frequency of 4 Hz, light pulses are shown in the periphery of the device at a frequency of 4 Hz, all the components of the haptic suit are actuated (e.g., vibrate), and the scent emitter emits a smell. Event 5 also includes provision of instructions for the user. The instructions are for getting on and off an inclined platform (hence, there must be an inclined platform near the user); the instructions are provided via sound emitted by the loudspeaker.

In the event 6, a dish of lentils with chicken is shown on the display and a commentary referring to the dish is emitted by the loudspeaker.

In the event 7, the following stimuli are simultaneously produced: a dish of azuki bean and red cabbage stew with orange sauce is shown on the display, the vibrating devices vibrate at a frequency of 4 Hz, light pulses are shown in the periphery of the device at a frequency of 4 Hz, all the components of the haptic suit are actuated (e.g., vibrate), the scent emitter emits a scent that smells like the stew, and between one and five drops of water go out of the open end 311 of the conduit of the taste stimulator. The event 7 also includes provision of instructions for the user. The instructions are for focusing on taste; the instructions are provided via sound emitted by the loudspeaker.

In the event 8, the following stimuli are simultaneously produced: a bathroom is shown on the display and the loudspeaker emits music. Event 8 also includes provision of instructions for the user. The instructions are for looking for a soap; the instructions are provided via sound emitted by the loudspeaker.

In the event 9, the following stimuli are simultaneously produced: a bathroom is shown on the display, the loudspeaker emits music, the vibrating devices vibrate at a frequency of 4 Hz, light pulses are shown in the periphery of the device at a frequency of 4 Hz, the scent emitter emits a scent that smells like a bathroom, and between one and five drops of water go out of the open end 311 of the conduit of the taste stimulator. Event 9 also includes provision of instructions for the user. The instructions are for looking for a toothbrush; the instructions are provided via sound emitted by the loudspeaker.

In the event 10, the following stimuli are simultaneously produced: a person is shown on the display, the loudspeaker emits music, the vibrating devices vibrate at a frequency of 4 Hz, light pulses are shown in the periphery of the device at a frequency of 4 Hz, all the components of the haptic suit are actuated (e.g., vibrate), the scent emitter emits a scent.

In the event 11, the following stimuli are simultaneously produced: a sea is shown on the display, the loudspeaker emits music, the vibrating devices vibrate at a frequency of 4 Hz, light pulses are shown in the periphery of the device at a frequency of 4 Hz, all the components of the haptic suit are actuated (e.g., vibrate), the scent emitter emits a scent that smells like a sea, and between one and five drops of water go out of the open end 311 of the conduit of the taste stimulator. The event 11 also includes provision of instructions for the user. The instructions are for hearing the sound of the sea and breathing at the pace of waves of the sea; the instructions are provided via sound emitted by the loudspeaker.

In the event 12, several images are shown on the display, wherein some images of the several images have been shown on the display earlier in the session and other images of the several images have not been shown on the display earlier in the session; and some images of the images shown on the display earlier in the session have been shown while simultaneously stimulating five senses of the user. For example, the following images may be shown on the display: a toothbrush, a soap, a comb, a glass, a rose, a geranium, lavender, a lemon, radishes, olive oil, azuki beans and a pomegranate. The event 12 also includes provision of instructions for the user. The instructions are to determine, for each image of the several images, if the respective image has been previously shown on the display during the session; the instructions are provided via sound emitted by the loudspeaker and/or via one or more images shown on the display.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A system (1) for performing a session of stimulation of five senses A, B, C, D and E of a human user, the session of stimulation comprising determined stimuli of the five senses and a determined stimulation time for each stimulus of the determined stimuli;
the system comprising stimulators of A, B, C, D and E, the stimulators of A, B, C, D and E being: a head-attachable display (11) for stimulating sight, a loudspeaker (12) for stimulating hearing, a tactile stimulator (13), a scent emitter (14) for stimulating smell and a taste stimulator (15);
the system (1) comprising processing means configured to actuate each one of the stimulators at determined actuation times to produce each stimulus of the determined stimuli at the respective stimulation time;
wherein the processing means are configured to actuate the stimulators of A, B, C, D and E to produce a simultaneous stimulation of A, B, C, D and E in first portions of a period of the session; and
wherein the processing means are configured to actuate at least one of the stimulators of A, B, C and D to stimulate at least one of A, B, C and D in each portion of the period of the session between two consecutive first portions, and are not configured to actuate the stimulator of E to stimulate E simultaneously with the stimulated at least one of A, B,C and D in each portion of the period of the session between two consecutive first portions.

2. The system (1) of claim 1, wherein the determined stimuli of the session comprises instructions for the user, the instructions being for using a sense in a determined manner in relation to an stimulus of the determined stimuli, the stimulus being different than instructions for the user; the system being configured to actuate an stimulator to produce, when the instructions for the user have ended, the stimulus different than instructions for the user.

3. The system (1) of any one of the previous claims, the tactile stimulator (13) comprising vibrating devices (132) for being attached to a human body, the system being configured to cause that the vibrating devices vibrate at a frequency of at least 3.5 Hz and at most 7.5 Hz.

4. The system (1) of any one of the previous claims, the system being configured to cause that the head-attachable display shows light pulses on the display, the light pulses having a frequency of at least 3.5 Hz and at most 7.5 Hz.

5. The system (1) of any one of the previous claims, the taste stimulator (15) comprising a conduit (31) with an open end (311), the conduit being for conveying an edible substance; the open end (311) being for exit of an edible substance and being introduceable in a mouth of a human user; wherein the taste stimulator (15) comprises a container (32) for an edible substance, the conduit (31) being connected to an interior of the container (32); the taste stimulator (15) comprising means (33) for moving an edible substance from the interior of the container (32) to the open end (311) of the conduit via the conduit.

6. The system (1) of claim 5, the system being configured to include, by using the means for moving an edible substance, an edible substance within the conduit when the processing means actuate the taste stimulator.

7. The system (1) of any one of the previous claims, the system being configured to provide, prior to starting a session, instructions to a user of the system (1), the instructions being for placing inside a mouth an open end of the taste stimulator, the open end being for exit of an edible substance; the system being configured to provide the instructions by at least one of:
• showing, on the head-attachable display (11), the instructions for placing the taste stimulator (15), and
• emitting, by the loudspeaker (12), sound comprising instructions for placing the taste stimulator (15).

8. The system (1) of any one of the previous claims, wherein, for producing a simultaneous stimulation of the five senses, the processing means are configured to actuate the taste stimulator (15) prior to actuating the other stimulators.

9. The system (1) of any one of the previous claims, the processing means being configured to leave a time of at least thirty seconds between two consecutive actuations of the taste stimulator (15).

10. The system (1) of any one of the previous claims, the system being configured to provide, prior to starting a session, instructions to a user of the system, the instructions being for placing the scent emitter (14) near the user; the system being configured to provide the instructions by at least one of:
• showing, on the head-attachable display (11), the instructions for placing the scent emitter (14) near the user, and/or
• emitting, by the loudspeaker (12), sound comprising the instructions for placing the scent emitter (14) near the user.

11. The system (1) of any one of the previous claims, the processing means being configured to leave a time of at least thirty seconds between two consecutive actuations of the scent emitter (14).

12. The system (1) of any one of the previous claims, the processing means being configured to leave a time of at least thirty seconds between two consecutive actuations of the scent emitter (14); wherein the processing means are configured to actuate the taste stimulator (15) to stimulate taste simultaneously with stimulation of smell produced by the latest actuation of the two consecutive actuations of the scent emitter (14).

13. The system (1) of any one of the previous claims, the session comprising showing several images on the display (11), wherein some images of the several images have been shown on the display (11) during the session and other images of the several images have not been shown on the display (11) during the session; and some images of the several images shown on the display (11) during the session have been shown while simultaneously stimulating five senses of the user; wherein the processing means are configured to provide instructions for the user to determine, for each image of the several images, if the respective image has been previously shown on the display (11) during the session.

14. A method for performing a session of stimulation of five senses A, B, C, D and E of a human user, the session of stimulation comprising determined stimuli of the five senses and a determined stimulation time for each stimulus of the determined stimuli;
the method comprising producing each stimulus of the determined stimuli at the respective stimulation time for the respective stimulus by actuating stimulators of the five senses, wherein in first portions of a period of the session a simultaneous stimulation of A, B, C, D and E is produced; and
wherein in each portion of the period of the session between two consecutive first portions of the session: at least one of A, B, C and D is stimulated, and E is not simultaneously stimulated with the stimulated at least one of A, B, C and D.
